# EUROPEAN PATENT SPECIFICATION

(11) **EP 3 033 986 B1**
(45) Date of publication and mention of the grant of the patent: **24.10.2018**
(21) Application number: 15824193.5
(22) Date of filing: 09.07.2015
(51) Int. Cl.: A61B 1/04, A61B 5/07, A61B 1/273, A61B 1/00

(54) **CONTROLLING EQUIPMENT AND SYSTEM OF CAPSULE ENDOSCOPE**
STEUERUNGSAUSRÜSTUNG UND -SYSTEM EINES KAPSELENDOSKOPS
ÉQUIPEMENT DE COMMANDE ET SYSTÈME D'ENDOSCOPE À CAPSULE

(30) Priority: 23.07.2014 CN 201410353836; 21.08.2014 CN 201410416049
(43) Date of publication of application: 22.06.2016
(73) Proprietor: Shenzhen Jifu Medical Technology Co. Ltd., Shenzhen, Guangdong 518057 (CN)
(72) Inventor: LI, Yi, Shenzhen Guangdong 518057 (CN); SUN, Ping, Shenzhen Guangdong 518057 (CN); DENG, Wenjun, Shenzhen Guangdong 518057 (CN); WANG, Jianping, Shenzhen Guangdong 518057 (CN)
(74) Representative: Zaboliene, Reda
(86) International application number: PCT/CN2015/083630
(87) International publication number: WO 2016/011895

(56) References cited:
- WO-A1-2009/107892
- WO-A1-2009/107892
- CN-A- 103 169 443
- CN-A- 103 405 211
- CN-A- 103 405 211
- CN-A- 104 146 676
- CN-U- 204 072 036
- DE-A1-102005 015 374
- DE-A1-102005 015 374
- US-A1- 2005 062 562
- US-A1- 2013 110 128

## Description

### TECHNICAL FIELD

The present invention relates to the field of medical devices, and in particular, to capsule endoscope control device and system.

### BACKGROUND

A capsule endoscope has the advantage of monitoring and diagnosis without pain and gash, which is gradually applied to clinical diagnosis for various diseases. When a capsule endoscope is taken by an examinee, the capsule endoscope enters the stomach of the examinee. Relevant data is collected by using a lens component or a sensor to make clinical diagnosis, thereby reducing clinical pain of the examinee.

When entering the stomach of the examinee, the capsule endoscope can freely operate. The position of the capsule endoscope is uncertain, and the collected data is arbitrary. In this case, whether the capsule endoscope collects all data in a target region of the stomach cannot be determined. Accordingly, it is hard to judge the condition of the examination region of the stomach. Therefore, how to effectively control positions and postures of the capsule endoscope in the human body is very important for picking up desired images for the stomach.
Chinese patent Publication 103405211 describes a capsule endoscope device having permanent magnet mounted on an arm which can be controlled to move the magnet and thus a capsule endoscope within a patient's body.
German Patent Publication 102005015374 describes a magnetically polarized capsule endoscope and a kit including a magnet control device.
PCT Publication WO2009/107892 describes an endoscope system using a capsule-type endoscope controlled by a rotating magnetic force.
US Patent Publication 2013/110128 describes a robotic magnetic guiding device for an intracorporeal object which includes a motor-driven positioning device having a maximum of three degrees of freedom.

### SUMMARY

The invention is defined in independent claim 1 and the dependent claims 2 - 13.

According to the embodiments of the present invention, by means of electrical control, the driving apparatus controls movement of the rotating apparatus and the movement arm, the permanent magnet disposed on the movement arm controls position and posture of the capsule endoscope in the human body, such that the capsule endoscope picks up complete images on the stomach, and accuracy and precision of medical diagnosis are improved.

### BRIEF DESCRIPTION OF DRAWINGS

FIG. 1 is a schematic structural view of a capsule endoscope control system according to an embodiment of the present invention;
FIGS. 2 and 3 are schematic structural views of a capsule endoscope control device according to an embodiment of the present invention;
FIG. 4 is a schematic structural rear view of a capsule endoscope control device according to an embodiment of the present invention;
FIG. 5 is a schematic sectional view taken along an S-S direction in FIG. 4;
FIG. 6 is a schematic structural view of a capsule endoscope control device covered with a cylinder according to an embodiment of the present invention;
FIG. 7 is a schematic structural view of a control apparatus in a capsule endoscope control system according to an embodiment of the present invention;
FIG. 8 is a schematic structural view of a driving apparatus in a capsule endoscope control device according to an embodiment of the present invention;
FIG. 9 is a schematic internal structural view of a capsule endoscope according to an embodiment of the present invention;
FIG. 10 is a schematic view of the side of the capsule endoscope with a lens close to an inner wall of the human body according to an embodiment of the present invention;
FIG. 11 is a schematic view of the side of the capsule endoscope without a lens close to an inner wall of the human body according to an embodiment of the present invention; and
FIG. 12 is a schematic view of a capsule endoscope picking up images for an inner wall of the human body in different postures of a permanent magnet according to an embodiment of the present invention.

### DETAILED DESCRIPTION

To make the objective, technical solution, and advantages of the present invention more clear, the following section describes the technical solutions of the present invention in combination with the accompanying drawings and embodiments. It should be understood that the embodiments described here are only exemplary ones for illustrating the present invention, and are not intended to limit the present invention.

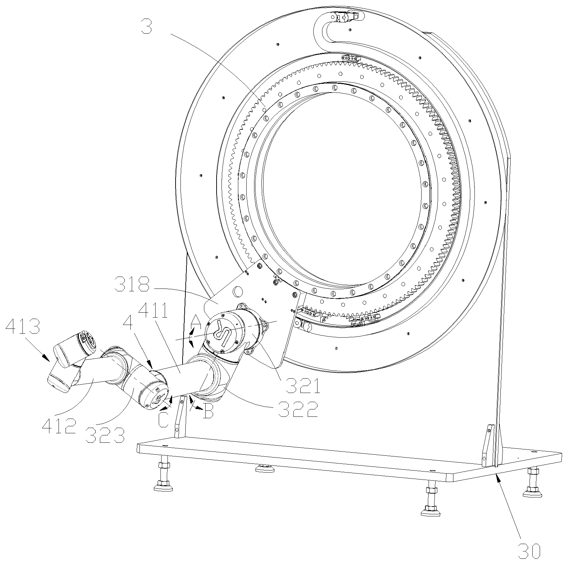

FIG. 1 illustrates a structure of a capsule endoscope control system according to an embodiment of the present invention. For ease of description, parts relevant to the embodiments of the present invention are only illustrated.

In the embodiment of the present invention, the capsule endoscope control system comprises a control apparatus 11 running on a terminal 1, and a capsule endoscope control device controllable by the control device 11.

The capsule endoscope control device comprises: a frame 30, a rotating apparatus 3, a movement arm 4, a permanent magnet 5, and a driving apparatus 2.

The rotating apparatus 3 is disposed on the frame 30.

The movement arm 4 is secured to the rotating apparatus 3, and a tail end of the movement arm 4 is provided with the permanent magnet 5.

The driving apparatus 2 is electrically connected to the rotating apparatus 3 and the movement arm 4, and configured to receive an external movement control instruction, drive the rotating apparatus 3, the movement arm 4 and the permanent magnet 5 to move, and control position and posture of the capsule endoscope in a human body via a magnetic force of the permanent magnet 5.

The control apparatus 11 running on the terminal 1 is communicated with the driving apparatus 2, and configured to provide an operation interface for a user, transmit a movement control instruction of the user to the driving apparatus 2, store and process image data collected by the capsule endoscope from the human body.

The control apparatus 11 is communicated with the capsule endoscope in a wireless manner, and may be communicated with the driving apparatus 2 in a wired manner or in a wireless manner.

In the embodiment of the present invention, the capsule endoscope control device employs an electrical control manner, and the driving apparatus 2 controls movement of the rotating apparatus 3 and the movement arm 4. Since the movement arm 4 is secured to the rotating apparatus 3, when the rotating apparatus 3 rotates, the movement arm 4 is driven to rotate as well, and meanwhile position and posture of the permanent magnet 5 are changed by regulating posture of the movement arm 4, thereby ensuring that the permanent magnet 5 is capable of traversing all positions in an examination region.

Referring to FIG. 1 to FIG. 3, the frame 30 comprises a base 301 supported on the ground, and a back plate 302 perpendicularly secured to the base 301 along a longitudinal direction. The rotating apparatus 3 is secured in an overlay manner to the back plate 302. The back plate 302 is provided with a through hole.

One end of the movement arm 4 is secured to a side surface of the rotating apparatus 3, and the other end of the movement arm 4 telescopically rotatably extends along a transverse direction.

The permanent magnet 5 is disposed at a tail end of the movement arm 4. When the capsule endoscope is taken by an examinee, the taken capsule endoscope is controlled by the permanent magnet 5.

The driving apparatus 2 is electrically connected to the rotating apparatus 3 and the movement arm 4, and configured to electrically control the rotating apparatus 3, the movement arm 4, and the permanent magnet 5.

There are multiple motors 32, which are separately disposed on the rotating apparatus 3 and the movement arm 4, and enables, according to the control instruction sent by the terminal 1 to the driving apparatus 2, the driving apparatus 2 to adjust the positions of the rotating apparatus 3 and the movement arm 4 so as to drive the permanent magnet 5 to reach the designated examination region.

The rotating apparatus 3 is in a hollow disk shape, and forms a receiving chamber 303 with a through hole opened on the back plate 302.

The receiving chamber 303 is configured to accommodate an examinee and is in a cylinder shape, is communicated with the rotating apparatus 3 and the back plate 302, and extends towards a stretching direction of the movement arm 4.

One end of the movement arm 4 is vertically secured to the rotating apparatus 3, and the other end of the movement arm 4 is movable on the outer side of the receiving chamber 303. There are multiple arm rods 41 that are sequentially connected.

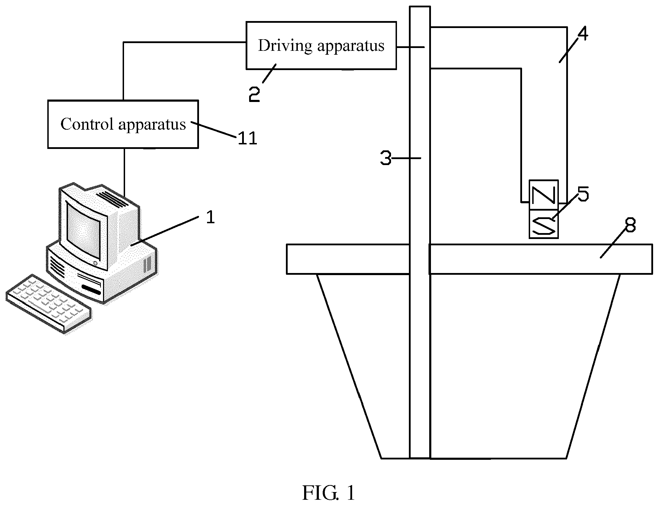

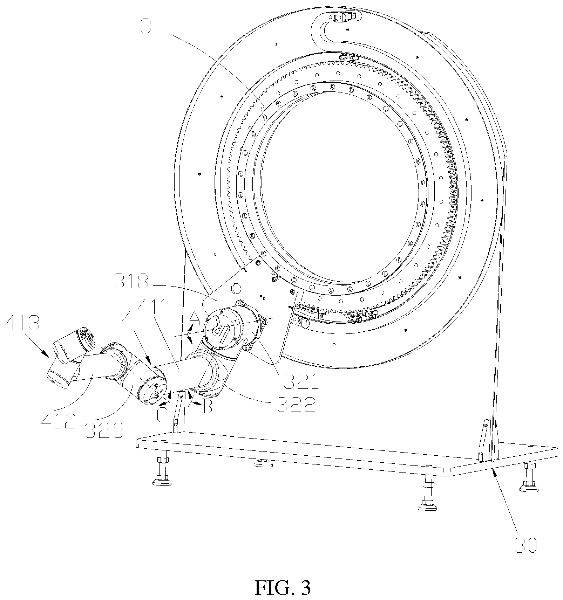

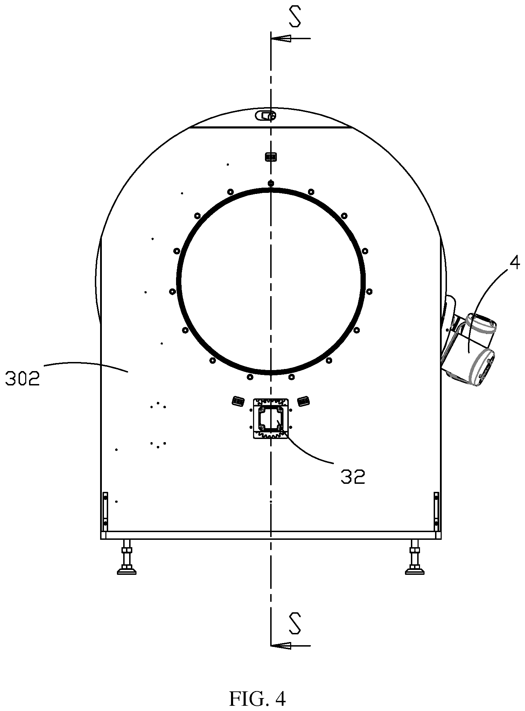

Referring to FIG. 2 to FIG. 5, the hollow disk-shaped rotating apparatus 3 comprises a driving wheel 311, a driven wheel 312, and a bearing 313.

The driving wheel 311 is securely disposed on one side at the bottom of the back plate 302, and a motor 32 connected to the driving wheel 311 is disposed on the other side of the back plate 302, wherein the motor 32 is configured to drive the driving wheel 311.

The driven wheel 312 is engaged with the driving wheel 311, and is rotatably connected to an outer ring of the bearing 313.

The bearing 313 is in hollow annular shape, and is secured to the back plate 302. The receiving chamber 303 is communicated with the bearing 313 and the back plate 302.

A guiding plate 314 is secured in an overlay manner to one side of the back plate 302. The guiding plate 314 is positioned on an outer ring at the bottom of the driven wheel 312.

An outer edge of the guiding plate 314 extends towards one side of the movement arm 4 to define a circular baffle 315, and a protrusion portion 316 being formed upwardly in a position close to the driven wheel 312 on an inner ring of the guiding plate 314. The baffle 315, the guiding plate 314, and the protrusion portion 316 define an annular guiding groove 317 therebetween.

When the rotating apparatus 3 drives the movement arm 4 to rotate, the activity space of a connection cable 401 of the movement arm 4 is provided. The connection cable 401 may be a signal cable and a power cable of the movement arm 4. To make the connection cable 401 tidy and beautiful, the connection cable 401 is sleeved with a drag chain 402. The drag chain 402 may be freely bent, and movable in the guiding groove 317.

An installation plate 318 is secured to a top surface of the driven wheel 312, and one end of the movement arm 4 is secured to the installation plate 318. When the driven wheel 312 rotates, the installation plate 318 rotates accordingly and drives the movement arm 4 to rotate.

The installation plate 318 is in a planar structure, and spans over the guiding groove 317. One end of the installation plate 318 is secured to the driven wheel 312, and the other end extends outside the circular baffle 315, such that the installation plate 318 will not disturb the drag chain 402 in the guiding groove 317 during the process of rotating.

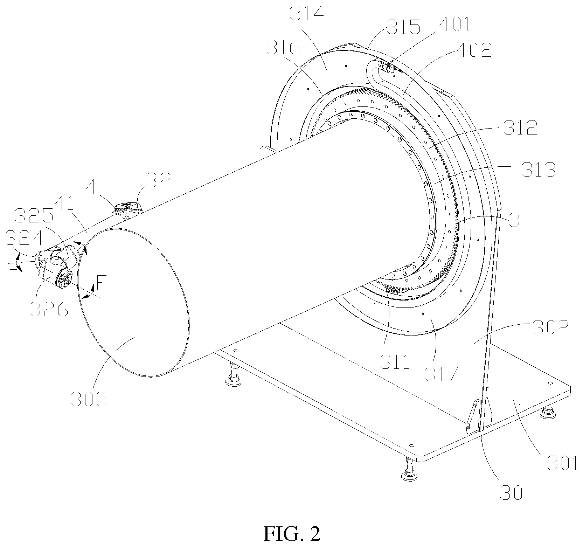

Referring to FIGS. 2 and 3, the movement arm 4 has at least two arm rods 41 connected with each other. The motor 32 is separately disposed between the arm rods 41, and between the arm rods 41 and the rotating apparatus 3.

When the arm rods 41 connect the motor 32, the movement arm 4 can stretch and rotate, and the stretching direction of the movement arm 4 is consistent with the receiving chamber 303. When an examinee is in the receiving chamber 303, the rotating apparatus 3 may rotate at 360 degrees, and drive the movement arm 4 to rotate round the receiving chamber 303, such that the examinee receives an all-round thorough examination. When an examination region is selected, the rotating apparatus 3 stops rotating, and the positions of the arm rods 41 are adjusted, such that the permanent magnet 5 traverses all positions in the examination region.

As a preferred embodiment of the present invention, the movement arm 4 may comprise a first arm rod 411, a second arm rod 412, and a third arm rod 413 that are connected to each other.

One end of the first arm rod 411 is secured to the rotating apparatus 3; one end of the second arm rod 412 is connected to the other end of the first arm rod 411; and one end of the third arm rod 413 is connected to the other end of the second arm rod 412, and the other end of the third arm rod 413 is connected to the permanent magnet 5. The motor 32 is separately disposed between the first arm rod 411 and the rotating apparatus 3, between the first arm rod 411 and the second arm rod 412, and between the second arm rod 412 and the third arm rod 413.

Specifically, the first arm rod 411 is secured to a side surface of the driven wheel 312 via the installation plate 318, and the motor 32 is disposed between the first arm rod 411 and the driven wheel 312. The motor 32 is separately disposed between the first arm rod 411 and the second arm rod 412, and between the second arm rod 412 and the third arm rod 413. The permanent magnet 5 is disposed on the third arm rod 413. Compared with the movement arm 4 using two arm rods 41, the movement arm 4 using three arm rods 41 can reduce the length of a single arm rod 41, and can more flexibly control the permanent magnet 5 disposed at a tail end.

As another embodiment of the present invention, the movement arm 4 has two arm rods: a first arm rod 411, and a second arm rod 412.

One end of the first arm rod 411 is secured to the rotating apparatus 3. One end of the second arm rod 412 is connected to the other end of the first arm rod 411, and the other end of the second arm rod 412 is connected to the permanent magnet 5. The motor 32 is separately disposed between the first arm rod 411 and the rotating apparatus 3, between the first arm rod 411 and the second arm rod 412, and between the second arm rod 412 and the permanent magnet 5. In this way, the third arm rod 413 in the above embodiment is replaced by using the length of the motor 32, the permanent magnet 5 is placed on the output end of the motor 32, and the same effects as the third arm rod 413 are achieved by using the length of the motor 32, thereby saving space.

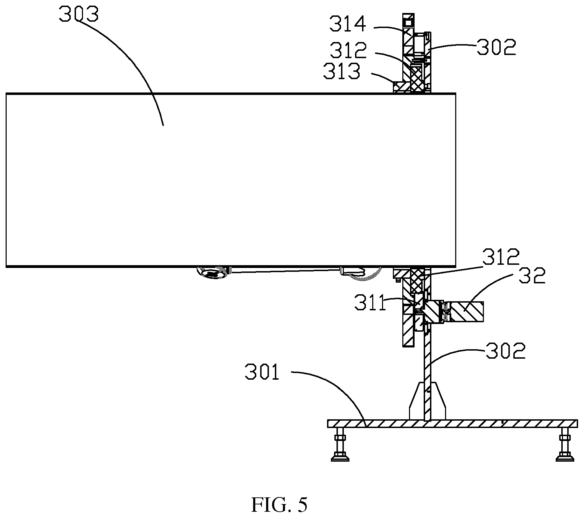

Referring to FIG. 6, to make capsule endoscope control system more beautiful, and ensure the security of an examinee, the rotating apparatus 3 and movement arm 4 can be covered in a cylinder 304. The cylinder 304 extends along the stretching direction of the movement arm 4, and the receiving chamber 303 penetrates through the cylinder 304 along an axial direction. The movement arm 4 using three arm rods 41 can make the diameter of the cylinder 304 smaller after the length of a single arm rod 41 is reduced. Under the same circumstances of traversing any position of the space of the cylinder 304, the cylinder 304 of the smaller size reduces the volume of entire capsule endoscope control system, which thus reduces space and saves manufacture costs.

Specifically, the motor 32 in the embodiment of the present invention is a servo motor.

The motor disposed between the first arm rod 411 and the driven wheel 312 comprises a first motor 321 and a second motor 322.

The first motor 321 is secured to the driven wheel 312 along a transverse direction, and the output end of the first motor 321 is connected to the second motor 322; and the second motor 322 is disposed along a longitudinal direction, and the output end of the second motor 322 is connected to the first arm rod 411. In this way, the first motor 321 rotates along a transverse axle A at 360 degrees, and the output end of the second motor 322 rotates at 360 degrees along a longitudinal axle B in a vertical plane of the first motor 321.

The motor disposed between the first arm rod 411 and the second arm rod 412 is a third motor 323. The third motor 323 is disposed along a longitudinal direction, and the output end of the third motor 323 is connected to the second arm rod 412. In this way, the output end of the third motor 323 rotates at 360 degrees along a longitudinal axle C in a vertical plane of the first arm rod 411.

The motors disposed between the second arm rod 412 and the third arm rod 413 are a fourth motor 324, a fifth motor 325, and a six motor 326 that are sequentially connected.

The fourth motor 324 is disposed along a longitudinal direction; the fifth motor 325 is disposed along a transverse direction, and connected to the output end of the fourth motor 324; the six motor 326 is disposed along a longitudinal direction, and connected to the output end of the fifth motor 325; and the permanent magnet 5 is disposed on the output end of the six motor 326. In this way, the fourth motor 324 rotates at 360 degrees along a longitudinal axle D in a vertical plane of the second arm rod 412, the fifth motor 325 rotates at 360 degrees along a transverse axle E in a vertical plane of the fourth motor 324, and the six motor 326 drives the permanent magnet 5 along a longitudinal axle F in a vertical plane vertical to the fifth motor 325 to rotate at 360 degrees.

Therefore, by adjusting angles of various motors, and flexibly controlling positions of arm rods 41 and permanent magnet 5, the permanent magnet 5 reaches entire position in the region of the cylinder 304.

A control apparatus 11 runs on the terminal 1, is configured to control running states of the entire capsule endoscope control system, and is communicated with the driving apparatus 2.

The operation interface of the control apparatus 11 comprises various function options. A user may directly operate on the operation interface such as, selecting working mode, or inputting other relevant information, for example, controlling position of the examination couch 8.

In addition, the control apparatus 11 internally contains instruction programs corresponding to various functions on the operation interface. After a user selects a function option, the control apparatus 11 sends the corresponding instruction to the driving apparatus 2. In this way, the user can control running of the capsule endoscope control system by using the control apparatus 11.

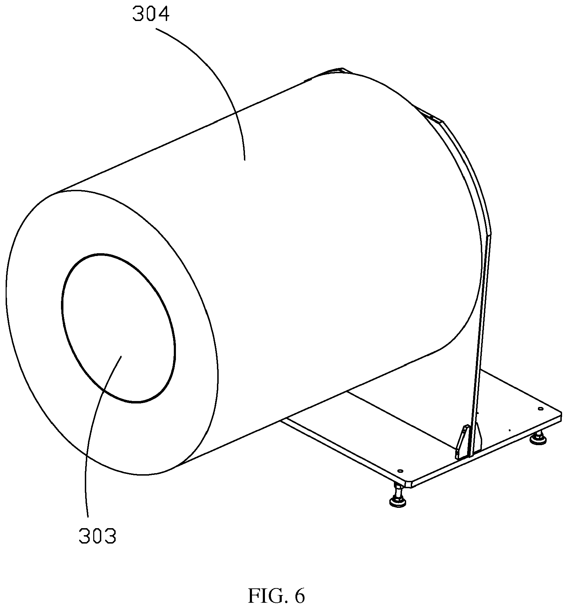

Referring to FIG. 7, the control apparatus 11 comprises: an instruction receiving unit 111, a data receiving unit 112, a data storing unit 113, and a data processing unit 114. The instruction receiving unit 111 is configured to transmit a control instruction input by the user to the driving apparatus 2.

The data receiving unit 112 is configured to receive image data collected and output by a capsule endoscope 7 from a human body.

The data storing unit 113 is configured to store the image data received by the data receiving unit 112.

The data processing unit 114 is configured to process, according to user's operations, the image data such as, browse, edit, and tag, stored by the data storing unit 113.

In an embodiment of the present invention, the terminal 1 is a computer, and may also be another upper-level computer capable of implementing the same functions.

The driving apparatus 2 comprises an input interface, a PLC module, a power module, and an output interface that are electrically connected; wherein: the input interface is configured to receive a movement control instruction input over the terminal 1, the PLC module is configured to perform an operation on the movement control instruction and output a movement control signal, the output interface is configured to output the movement control signal to the rotating apparatus 3 or the movement arm 4, and the power module is configured to supply power to the PLC module.

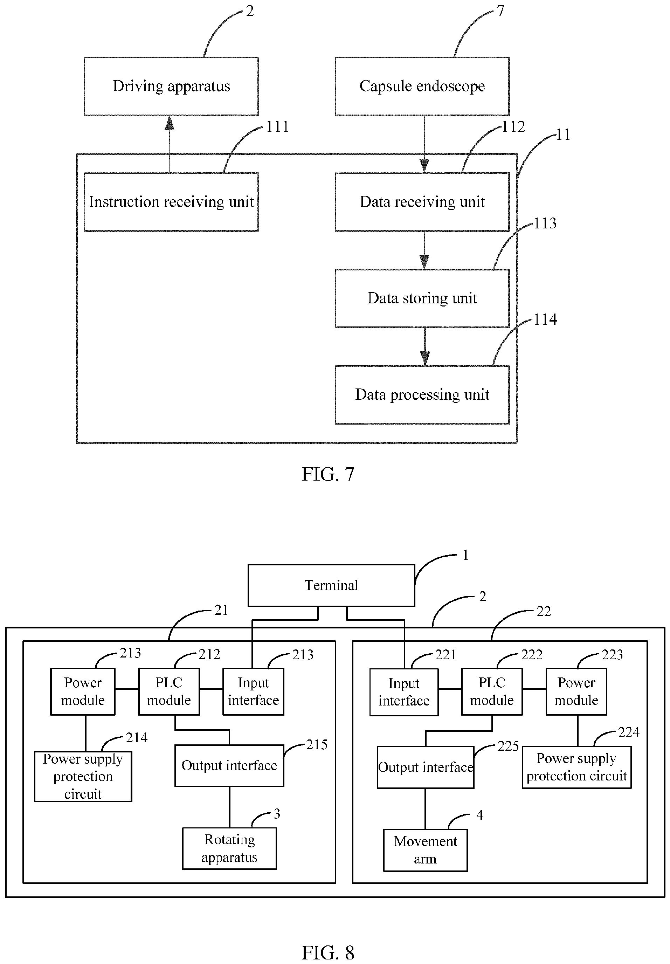

Referring to FIG. 8, the driving apparatus 2 is divided into a rotating apparatus driving module 21, and a movement arm driving module 22. The motor driving the driving wheel 311 is a rotating apparatus motor, and the motor disposed on each arm rod of the movement rod 4 is a movement arm motor.

Specifically, the rotating apparatus driving module 21 internally comprises: an input interface 211, a PLC module 212, a power supply module 213, a power supply protection circuit 214, and an output interface 215.

The input interface 211 is configured to receive instruction information transmitted by the terminal 1, and transmit the instruction information to the PLC module 212. The PLC module 212 internally stores logical operation programs. After receiving an input signal, the PLC module 212 runs the corresponding program according to the input signal, generates an output signal, and finally transmits the output signal to the output interface 215. The output interface 215 transmits the output signal to the rotating apparatus 3 to control rotation of the motor in the rotating apparatus 3.

The power supply module 213 is configured to supply power to the PLC module 212.

The power supply protection circuit 214 is configured to protect the power supply module 213 to prevent exceptions of over-voltage, over-temperature, and over-current.

The movement arm driving module 22 internally comprises: an input interface 221, a PLC module 222, a power supply module 223, a power supply protection circuit 224, and an output interface 225.

The input interface 221 is configured to receive instruction information transmitted by the terminal 1, and transmit the instruction information to the PLC module 222.

The PLC module 222 internally stores logical operation programs. After receiving an input signal, the PLC module 222 runs the corresponding program according to the input signal, and finally transmits an output signal to the output interface 225. The output interface 225 transmits the output signal to the movement arm 4 to control the movement arm 4 to implement corresponding movement.

The power supply module 223 is configured to supply power to the PLC module 222.

The power supply protection circuit 224 is configured to protect the power supply module 223 to prevent exceptions of over-voltage, over-temperature, and over-current.

The output signal transmitted by the rotating apparatus driving module 21 to the rotating apparatus 3 comprises: an angular velocity and rotation angle information of the rotation of the rotating apparatus motor. For example, when a pulse signal is selected as the output signal, the pulse frequency of the pulse signal and the number of pulse signals may be used to correspond to the angular velocity and the rotation angle according to an agreement. After receiving a signal, the rotating apparatus motor rotates at a corresponding angle at a corresponding angular velocity. The rotating apparatus 3 may be subjected to 360 degrees rotation.

The movement arm driving module 22 may transmit a signal to any motor on the movement arm 4. Under driving of the motor, each arm rod may move up, down, left, and right. Therefore, the movement arm 4 moves to a designated position within a specific range under cooperation of the arm rods thereof.

The output signal transmitted by the movement arm driving module 22 comprises such information as movement direction and distance of the arm rods. For example, when a pulse signal is selected as the output signal, the pulse frequency of the pulse signal and the number of pulse signals may be used to correspond to the movement direction and distance of arm rods according to an agreement. Under the cooperative action of the arm rods of the movement rod 4, the movement rod 4 may change the position of the permanent magnet 5 through a series of operations such as shrink and move, and may also control the permanent magnet 5 through a rotation operation to rotate at 360 degrees with a peripheral point as a center of circle, or to self-rotate at 360 degrees with the center of the permanent magnet 5 as an axle. Through the above operations, the movement rod 4 changes the position and posture of the permanent magnet 5.

Referring to FIG. 1, as a preferable embodiment of the present invention, to facilitate examination on an examinee, an examination couch 8 is disposed below the rotating apparatus 3 and the movement arm 4. The examination couch 8 is configured to support an examinee, and may be movably pushed.

The examination couch 8 horizontally passes through the rotating apparatus 3 and is thus placed, and is configured to accommodate the examinee taking a controllable capsule endoscope in the body. After taking a capsule endoscope, the examinee lies down on the examination couch 8. Under the control of the capsule endoscope control system, the permanent magnet 5 may reach a series of different positions around the examinee and stay in different postures. Because the magnetic material in the capsule endoscope is subjected to a magnetic force, the position of the permanent magnet 5 may change under traction of the magnetic force, the movement direction of the capsule endoscope may also change, and finally the capsule endoscope may move to the position corresponding to the permanent magnet 5. If the posture of the permanent magnet 5 changes, the posture of the capsule endoscope may correspondingly change.

The method for controlling capsule endoscope in the body by using the permanent magnet 5 in the capsule endoscope control system is specifically described hereinafter.

As illustrated in FIG. 9, the capsule endoscope 7 in the embodiment of the present invention comprises: a capsule housing 71, a lens hood 72, and a lens 73 internally disposed therein, an LED light source component 74, a magnet 75, and a circuit board 76, a battery 77, and an antenna 78.

Only a group of lens 73 is selected in the embodiment of the present invention, the lens hood 72 is disposed on a periphery of the lens 73, the battery 77 is connected to the circuit board 76, and the antenna 78 is connected to the circuit board 76. The selected magnetic material in the embodiment of the present invention is magnet 75 disposed on a periphery of the battery 77. The magnet 75 is subjected to a magnetic force in the magnetic field to change the movement state of the capsule endoscope 7.

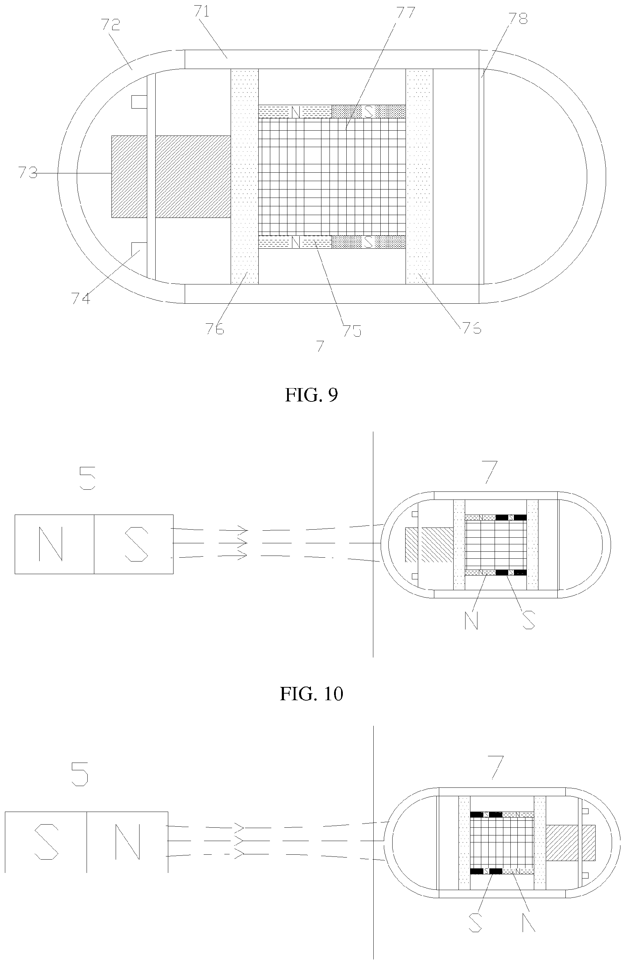

As illustrated in FIG. 10, the side of the capsule endoscope 7 with a lens needs to be disposed close to the stomach wall when images of the stomach wall in a specific position need to be clearly picked up.

The capsule endoscope control system controls the permanent magnet 5 to be located at a specific position close to the stomach in the human body, and the S pole of the permanent magnet 5 points to the human body, and the N pole points to the opposite. Under traction of the magnetic force, the capsule endoscope 7 moves towards the direction of the S pole of the permanent magnet 5. When moving to the stomach wall close to the S pole of the permanent magnet, the capsule endoscope 7 stops moving. Because the magnetic pole of the magnet has the features of "like charges repel each other while unlike charges attract", the N pole of the permanent magnet 5 points to the stomach wall, and the N pole points to the opposite. Accordingly, the side of the capsule endoscope 7 with a lens is disposed close to the stomach wall, and the side without a lens is disposed close to the opposite. At this moment, images in a series of the corresponding positions of the stomach wall can be picked up in a short distance. With the same method, the capsule endoscope control system controls the permanent magnet 5 to be located at different positions close to the stomach in the human body, and thus images in a series of the corresponding positions of the stomach wall can be precisely picked up.

As illustrated in FIG. 11, the side of the capsule endoscope 7 without a lens needs to be disposed close to the stomach wall when a large range of images need to be picked up.

The specific implementing method is as follows: the capsule endoscope control system controls the permanent magnet 5 to be located at a specific position close the stomach in the human body, the N pole points to the human body, and the S pole points to the opposite. Under traction of the magnetic force, the capsule endoscope 7 moves towards the direction of the N pole of the permanent magnet. When moving to the stomach wall close to the N pole of the permanent magnet, the capsule endoscope 7 stops moving, the S pole of the permanent magnet 5 points to the stomach wall, and the N pole points to the opposite. Correspondingly, the side of the capsule endoscope 7 without a lens is disposed close to the stomach wall, and the side with a lens is disposed close to the opposite. At this moment, images within a large range of the stomach wall of the human body can be picked up in a remote distance. With the same method, the capsule endoscope control system controls the permanent magnet 5 to be located in different positions close to the stomach in the human body, and thus images in a series of the corresponding positions of the stomach wall can be picked up.

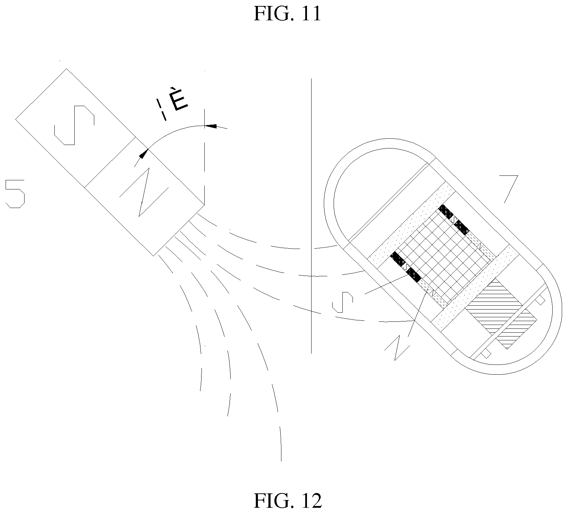

FIG. 12 illustrates a method for changing postures of the capsule endoscope 7 by changing postures of the permanent magnet 5.

The capsule endoscope control system controls the permanent magnet 5 to be located in a specific position that forms an angle θ (0 < θ < 180) with the human body. The N pole points to the human body at an angle θ, and the S pole points to the opposite. Under traction of the magnetic force, the capsule endoscope 7 finally moves to the position as illustrated in FIG. 12 , the side of the capsule endoscope 7 without a lens points to the stomach wall at the angle θ, and the side with a lens points to the opposite. In this way, the permanent magnet 5 is controlled in different postures by changing the angle θ, which can make the capsule endoscope 7 in different postures, such that the image can be picked up at any angle in the original position.

According to the embodiments of the present invention, by means of electrical control, the driving apparatus controls movement of the rotating apparatus and the movement arm, the permanent magnet disposed on the movement arm controls position and posture of the capsule endoscope in the human body, such that the capsule endoscope picks up complete images on the stomach, and accuracy and precision of medical diagnosis are improved.

Described above are merely preferred embodiments of the present invention, but are not intended to limit the present invention.

## Claims

1. A capsule endoscope control device (11), comprising:
a frame (30) comprising a base (301) supported on the ground, and a back plate (302) perpendicularly secured to
the base (301) along a longitudinal direction;
a rotating apparatus (3), disposed on the frame (30) and secured to the back plate;
a movement arm (4), secured to the rotating apparatus (3), a tail end of the movement arm (4) being provided with a permanent magnet (5); and
a driving apparatus (2), electrically connected to the rotating apparatus (3) and the movement arm (4), and configured to receive an external movement control instruction, drive the rotating apparatus, the movement arm [4] and the permanent magnet (5) to move, and control position and posture of the capsule endoscope (7) in a human body via a magnetic force of the permanent magnet (5);
**characterised in that**:
the rotating apparatus comprises:
a driving wheel (311), securely disposed on one side of the back plate (302);
a driving wheel motor, securely disposed on the other side of the back plate (302), and electrically connected to the driving wheel;
a bearing (313), secured to the back plate (302); and
a driven wheel (312), engaged with the driving wheel (311), and rotatably connected to an outer ring of the bearing.

2. The capsule endoscope control device according to claim 1, wherein the back
plate (302) is provided with a through hole;
the bearing (313) is in a hollow annular shape;
wherein the through hole is communicated with a hollow position of the bearing (313) to define a receiving chamber (303) for accommodating an examinee; and
the movement arm (4) is positioned on an outer side of the receiving chamber (303).

3. The capsule endoscope control device according to claim 1, wherein a guiding plate (314) is secured in an overlay manner to one side of the back plate (302);
wherein the guiding plate (314) is positioned on an outer ring at a bottom side of the driven wheel (312); and
an outer edge of the guiding plate (314) extends towards one side of the movement arm (4) to define a circular baffle (315), a protrusion portion (316) being formed upwardly in a position close to the driven wheel on an inner ring of the guiding plate (314);
the baffle (315), the guiding plate (314), and the protrusion portion (316) defining an annular guiding groove (317);
the guiding groove (317) being internally provided with a connection cable (401) of the movement arm (4).

4. The capsule endoscope control device according to claim 1, wherein an installation plate (318) is secured to a top surface of the driven wheel (312), and one end of the movement arm (4) is secured to the installation plate (318).

5. The capsule endoscope control device according to claim 1, wherein the movement arm (4) comprises:
a first arm rod (411), one end of the first arm rod being secured to the rotating apparatus (3); and a second arm rod (412), one end of the second arm rod (412) being connected to the other end of the first arm rod (411), and the other end of the second arm rod being connected to the permanent magnet (5);
wherein a motor is separately disposed between the first arm rod (411) and the rotating apparatus (3), between the first arm rod (411) and the second arm rod (412), and between the second arm rod (412) and the permanent magnet (5).

6. The capsule endoscope control device according to claim 1, wherein the movement arm (4) comprises:
a first arm rod (411), one end of the first arm rod (411) being secured to the rotating apparatus (3);
a second arm rod (412), one end of the second arm rod being connected to the other end of the first arm rod (411); and
a third arm rod (413), one end of the third arm rod (413) being connected to the other end of the second arm rod (412), and the other end of the third arm rod (413) being connected to the permanent magnet (5);
wherein a motor is separately disposed between the first arm rod (411) and the rotating apparatus (3), between the first arm rod (411) and the second arm rod (412), and between the second arm rod and the third arm rod (413).

7. A capsule endoscope control system, comprising a capsule endoscope control device (11) as claimed in claim 1 and
a control apparatus, communicated with the driving apparatus (2), and configured to provide an operation interface for a user, transmit a movement control instruction of the user to the driving apparatus (2), store and process image data collected by the capsule endoscope (7) from the human body.

8. The capsule endoscope control system according to claim 7, wherein the back plate (302) is provided with a through hole;
the bearing (313) is in a hollow annular shape;
wherein the through hole is communicated with a hollow position of the bearing to define a receiving chamber (303) for accommodating an examinee and the movement arm (4) is positioned on an outer side of the receiving chamber (303).

9. The capsule endoscope control system according to claim 7, wherein a guiding plate is secured in an overlay manner to one side of the back plate (302);
wherein the guiding plate (314) is positioned on an outer ring at a bottom side of the driven wheel (312); and
an outer edge of the guiding plate (314) extends towards one side of the movement arm to define a circular baffle (315), a protrusion portion (316) being formed upwardly in a position close to the driven wheel on an inner ring of the guiding plate (314);
the baffle (315), the guiding plate (314), and the protrusion portion (316) defining an annular guiding groove (317);
the guiding groove (317) being internally provided with a connection cable (401) of the movement arm (4).

10. The capsule endoscope control system according to claim 7, wherein an installation plate (318) is secured to a top surface of the driven wheel (312), and one end of the movement arm (4) is secured to the installation plate (318).

11. The capsule endoscope control system according to claim 7, wherein the movement arm comprises:
a first arm rod (411), one end of the first arm rod (411) being secured to the rotating apparatus (3);
a second arm rod (412), one end of the second arm rod (412) being connected to the other end of the first arm rod (411), and the other end of the second arm rod (412) being connected to the permanent magnet (5);
wherein a motor is separately disposed between the first arm rod (411) and the rotating apparatus (3), between the first arm rod (411) and the second arm rod (412), and between the second arm rod (412) and the permanent magnet (5).

12. The capsule endoscope control system according to claim 7, wherein the movement arm (4) comprises:
a first arm rod (411), one end of the first arm rod being secured to the rotating apparatus (3);
a second arm rod (412), one end of the second arm rod (412) being connected to the other end of the first arm rod (411); and
a third arm rod (413), one end of the third arm rod (413) being connected to the other end of the second arm rod (412), and the other end of the third arm rod (413) being connected to the permanent magnet (5);
wherein a motor is separately disposed between the first arm rod (411) and the rotating apparatus (3), between the first arm rod (411) and the second arm rod (412), and between the second arm rod (412) and the third arm rod (413).

13. The capsule endoscope control system according to claim 7, wherein the control apparatus comprises:
an instruction receiving unit (111), configured to transmit the movement control instruction input by the user to the driving apparatus (2);
a data receiving unit (113), configured to receive the image data collected and output by the capsule endoscope (7) from the human body;
a data storing unit (113), configured to store the image data received by the data receiving unit; and
a data processing unit (114), configured to process, according to user's operations, the image data stored by the data storing unit (113).

## Patentansprüche

1. Kapselendoskop-Steuervorrichtung (11), umfassend:
einen Rahmen (30), der eine auf dem Boden abgestützte Basis (301) und eine Rückplatte (302) umfasst, die senkrecht an der Basis (301) entlang einer Längsrichtung befestigt ist;
eine Drehvorrichtung (3), die an dem Rahmen (30) angeordnet und an der Rückplatte befestigt ist;
einen Bewegungsarm (4), der an der Drehvorrichtung (3) befestigt ist, wobei ein hinteres Ende des Bewegungsarms (4) mit einem Permanentmagneten (5) versehen ist; und
eine Antriebsvorrichtung (2), die elektrisch mit der Drehvorrichtung (3) und dem Bewegungsarm (4) verbunden ist und so konfiguriert ist, um eine externe Bewegungssteueranweisung zu empfangen, die Drehvorrichtung, den Bewegungsarm (4) und den Permanentmagneten (5) zu bewegen und die Position und Haltung des Kapselendoskops (7) im menschlichen Körper über eine Magnetkraft des Permanentmagneten (5) zu kontrollieren;
**dadurch gekennzeichnet, dass**:
die Drehvorrichtung umfasst:
ein Antriebsrad (311), das sicher auf einer Seite der Rückplatte (302) angeordnet ist; einen Antriebsradmotor, der fest auf der anderen Seite der Rückplatte (302) angeordnet und elektrisch mit dem Antriebsrad verbunden ist;
ein Lager (313), das an der Rückplatte (302) befestigt ist; und
ein angetriebenes Rad (312), das mit dem Antriebsrad (311) in Eingriff steht und drehbar mit einem äußeren Ring des Lagers verbunden ist.

2. Kapselendoskop-Steuervorrichtung nach Anspruch 1, wobei die Rückplatte (302) mit einem Durchgangsloch versehen ist;
das Lager (313) ist in einer hohlen ringförmigen Form;
wobei das Durchgangsloch mit einer hohlen Position des Lagers (313) verbunden ist, um eine Aufnahmekammer (303) zum Unterbringen eines Probanden zu definieren; und der Bewegungsarm (4) ist an einer Außenseite der Aufnahmekammer (303) positioniert.

3. Kapselendoskop-Steuervorrichtung nach Anspruch 1, wobei eine Führungsplatte (314) in einer Überlagerungsart an einer Seite der Rückplatte (302) befestigt ist;
wobei die Führungsplatte (314) an einem äußeren Ring an einer Bodenseite des angetriebenen Rads (312) positioniert ist; und
eine äußere Kante der Führungsplatte (314) erstreckt sich zur einen Seite des Bewegungsarms (4), um eine kreisförmige Ablenkplatte (315) zu definieren, wobei ein Vorsprungabschnitt (316) in einer Position nahe dem angetriebenen Rad an der Innenseite des Rings der Führungsplatte (314) nach oben ausgebildet ist;
die Ablenkplatte (315), die Führungsplatte (314) und der Vorsprungsabschnitt (316) definieren eine ringförmige Führungsnut (317);
die Führungsnut (317) ist innen mit einem Verbindungskabel (401) des Bewegungsarms (4) versehen.

4. Kapselendoskop-Steuervorrichtung nach Anspruch 1, wobei eine Installationsplatte (318) an einer oberen Oberfläche des angetriebenen Rads (312) befestigt ist und ein Ende des Bewegungsarms (4) an der Installationsplatte (318) befestigt ist.

5. Kapselendoskop-Steuervorrichtung nach Anspruch 1, wobei der Bewegungsarm (4) umfasst:
eine erste Armstange (411), wobei ein Ende der ersten Armstange an der Drehvorrichtung (3) befestigt ist; und eine zweite Armstange (412), wobei ein Ende der zweiten Armstange (412) mit dem anderen Ende der ersten Armstange (411) verbunden ist und das andere Ende der zweiten Armstange mit dem Permanentmagneten (5) verbunden ist;
wobei ein Motor getrennt zwischen der ersten Armstange (411) und der Drehvorrichtung (3) zwischen der ersten Armstange (411) und der zweiten Armstange (412) und zwischen der zweiten Armstange (412) und dem Permanentmagnet (5) angeordnet ist.

6. Kapselendoskop-Steuervorrichtung nach Anspruch 1, wobei der Bewegungsarm (4) umfasst:
eine erste Armstange (411), wobei ein Ende der ersten Armstange (411) an der Drehvorrichtung (3) befestigt ist; eine zweite Armstange (412), wobei ein Ende der zweiten Armstange mit dem anderen Ende der ersten Armstange (411) verbunden ist; und
eine dritte Armstange (413), wobei ein Ende der dritten Armstange (413) mit dem anderen Ende der zweiten Armstange (412) verbunden ist und das andere Ende der dritten Armstange (413) mit dem Permanentmagnet (5) verbunden ist;
wobei ein Motor getrennt zwischen der ersten Armstange (411) und der Drehvorrichtung (3) zwischen der ersten Armstange (411) und der zweiten Armstange (412) und zwischen der zweiten Armstange und der dritten Armstange (413) angeordnet ist.

7. Kapselendoskop-Steuersystem, umfassend eine Kapselendoskop-Steuervorrichtung (11) nach Anspruch 1 und eine Steuervorrichtung, die mit der Antriebsvorrichtung (2) kommuniziert und so konfiguriert ist, um eine Bedienungsschnittstelle für den Benutzer bereitzustellen, um eine Bewegungssteueranweisung des Benutzers an die Antriebsvorrichtung (2) zu übertragen, Bilddaten, die von dem Kapselendoskop (7) vom menschlichen Körper gesammelt werden, speichern und verarbeiten.

8. Kapselendoskop-Steuersystem nach Anspruch 7, wobei die Rückplatte (302) mit einem Durchgangsloch versehen ist;
das Lager (313) ist in einer hohlen ringförmigen Form;
wobei das Durchgangsloch mit einer hohlen Position des Lagers in Verbindung steht, um eine Aufnahmekammer (303) zum Aufnehmen eines Probanden zu definieren, und der Bewegungsarm (4) an einer Außenseite der Aufnahmekammer (303) positioniert ist.

9. Kapselendoskop-Steuersystem nach Anspruch 7, wobei eine Führungsplatte in einer Überlagerungsmethode an einer Seite der Rückplatte (302) befestigt ist;
wobei die Führungsplatte (314) an einem äußeren Ring an einer Bodenseite des angetriebenen Rads (312) positioniert ist; und
eine äußere Kante der Führungsplatte (314) erstreckt sich zur einen Seite des Bewegungsarms, um eine kreisförmige Ablenkplatte (315) zu definieren, wobei ein Vorsprungabschnitt (316) in einer Position nahe dem angetriebenen Rad an einem inneren Ring der Führungsplatte (314) nach oben ausgebildet ist;
die Ablenkplatte (315), die Führungsplatte (314) und der Vorsprungsabschnitt (316) definieren eine ringförmige Führungsnut (317);
die Führungsnut (317) ist innen mit einem Verbindungskabel (401) des Bewegungsarms (4) versehen.

10. Kapselendoskop-Steuersystem nach Anspruch 7, wobei eine Installationsplatte (318) an einer oberen Oberfläche des angetriebenen Rads (312) befestigt ist und ein Ende des Bewegungsarms (4) an der Installationsplatte (318) befestigt ist.

11. Kapselendoskop-Steuersystem nach Anspruch 7, wobei der Bewegungsarm umfasst:
eine erste Armstange (411), wobei ein Ende der ersten Armstange (411) an der Drehvorrichtung (3) befestigt ist; eine zweite Armstange (412), wobei ein Ende der zweiten Armstange (412) mit dem anderen Ende der ersten Armstange (411) verbunden ist und das andere Ende der zweiten Armstange (412) mit dem Permanentmagnet (5) verbunden ist;
wobei ein Motor getrennt zwischen der ersten Armstange (411) und der Drehvorrichtung (3) zwischen der ersten Armstange (411) und der zweiten Armstange (412) und zwischen der zweiten Armstange (412) und dem Permanentmagnet (5) angeordnet ist.

12. Kapselendoskop-Steuersystem nach Anspruch 7, wobei der Bewegungsarm (4) umfasst:
eine erste Armstange (411), wobei ein Ende der ersten Armstange an der Drehvorrichtung (3) befestigt ist; eine zweite Armstange (412), wobei ein Ende der zweiten Armstange (412) mit dem anderen Ende der ersten Armstange (411) verbunden ist; und
eine dritte Armstange (413), wobei ein Ende der dritten Armstange (413) mit dem anderen Ende der zweiten Armstange (412) verbunden ist und das andere Ende der dritten Armstange (413) mit dem Permanentmagnet (5) verbunden ist;
wobei ein Motor getrennt zwischen der ersten Armstange (411) und der Drehvorrichtung (3) zwischen der ersten Armstange (411) und der zweiten Armstange (412) und zwischen der zweiten Armstange (412) und der dritten Armstange (413) angeordnet ist.

13. Kapselendoskop-Steuersystem nach Anspruch 7, wobei die Steuervorrichtung aufweist:
eine Anweisungsempfangseinheit (111), die so konfiguriert ist, um den Bewegungssteuerbefehl, der von dem Benutzer eingegeben wird, an die Antriebsvorrichtung (2) zu übertragen;
eine Datenempfangseinheit (113), die so konfiguriert ist, um die Bilddaten zu empfangen, die von dem Kapselendoskop (7) vom menschlichen Körper gesammelt und ausgegeben werden;
eine Datenspeichereinheit (113), die so konfiguriert ist, um die von der Datenempfangseinheit empfangenen Bilddaten zu speichern; und eine Datenverarbeitungseinheit (114), die so konfiguriert ist, dass sie gemäß den Operationen des Benutzers die von der Datenspeichereinheit (113) gespeicherten Bilddaten verarbeitet.

## Revendications

1. Un appareil de commande d'endoscope à capsule (11), comprenant:
un châssis (30) comprenant une base (301) appuyée au sol et une plaque arrière (302) fixée perpendiculairement sur la base (301) le long d'un axe longitudinal;
un dispositif de rotation (3), placé sur le châssis (30) et fixé sur la plaque arrière;
un bras mobile (4), fixé sur le dispositif de rotation (3), une queue du bras mobile (4) doté d'un aimant permanent (5); et
un dispositif d'entraînement (2), raccordé électriquement au dispositif de rotation (3) et au bras mobile (4) et configuré pour recevoir un module externe de commande de mouvement, entraîner le dispositif de rotation, le bras mobile (4) et l'aimant permanent (5) pour actionner et commander la position et la posture de cet endoscope à capsule (7) dans un corps humain, par l'intermédiaire d'une force magnétique de l'aimant permanent (5);
**caractérisé en ce que**:
le dispositif de rotation comprenne :
une roulette d'entraînement (311) solidement fixée sur l'un des côtés de la plaque arrière (302);
un moteur d'entraînement de la roulette d'entraînement, solidement fixé sur l'autre côté de la plaque arrière (302), et raccordé électriquement à l'roulette d'entraînement ;
un palier (313), fixé sur la plaque arrière (302) ; et
Une roulette entraînée (312) couplée à la roulette d'entraînement (311) et raccordée de façon à pouvoir tourner à l'anneau extérieur du palier.

2. L'appareil de commande d'endoscope à capsule, conformément à la revendication 1, est doté dans la plaque arrière (302) d'un orifice de passage;
le palier (313) a une forme annulaire creuse;
dans laquelle, l'orifice de passage est mis en contact avec une position creuse du palier (313) afin de déterminer une chambre de réception (303) pour héberger un candidat; et
le bras mobile (4) est placé sur un côté externe de la chambre de réception (303).

3. L'appareil de commande d'endoscope à capsule, conformément à la revendication 1, dans lequel une plaque directrice (314) est fixée de manière superposée sur un des côtés de la plaque arrière (302);
dans laquelle plaque directrice (314) est placée sur un anneau extérieur, sur le fond de la roulette entraînée (312); et
un bord extérieur de la plaque directrice (314) se prolonge en direction de l'un des côtés du bras mobile (4) pour circonscrire un déflecteur circulaire (315) formant ainsi une partie protubérante (316) pointée vers le haut, dans une position proche de la roulette entraînée, sur un anneau interne de la plaque directrice (314);
le déflecteur (315), la plaque directrice (314) et la partie protubérante (316) délimitent une rainure directrice annulaire (317);
la rainure directrice (317) est dotée à l'intérieur, d'un câble de raccordement (401) au bras mobile (4).

4. Conformément à la revendication 1, l'appareil de commande d'endoscope à capsule, dans lequel une plaque d'installation (318) est fixée sur la surface supérieure de la roulette entraînée (312), et l'une des extrémités du bras mobile (4) est fixée sur la plaque d'installation (318).

5. Conformément à la revendication 1, l'appareil de commande de l'endoscope à capsule, dans lequel le bras mobile (4) comprend :
une première tige de bras (411), fixée au dispositif de rotation (3) et une deuxième tige de bras (412) avec l'une des extrémités de la deuxième tige de bras (412) raccordée à l'autre extrémité de la première tige de bras (411) et l'autre extrémité de la deuxième tige de bras étant raccordée à l'aimant permanent (5);
dans ce dispositif, un moteur est place séparément entre la première tige de bras (411) et le dispositif de rotation (3), entre la première tige de bras (411) et la deuxième tige de bras (412), et entre la deuxième tige de bras (412) et l'aimant permanent (5).

6. Conformément à la revendication 1, l'appareil de commande de l'endoscope à capsule, dans lequel le bras mobile (4) comprend:
une première tige de bras (411) dont l'une des extrémités est fixée au dispositif de rotation (3) ;
une deuxième tige de bras (412) dont l'une des extrémités est raccordée à l'autre extrémité de la première tige de bras (411); et
une troisième tige de bras (413), dont l'une (413) des extrémités est raccordée à l'autre extrémité de la deuxième tige de bras (412), et l'autre extrémité de la troisième tige de bras (413) étant raccordée à l'aimant permanent (5);
dans ce dispositif, un moteur est placé séparément, entre la première tige de bras (411) et le dispositif de rotation (3) entre la première tige de bras (411) et la deuxième tige de bras (412) et entre la deuxième tige de bras et la troisième tige de bras (413).

7. Un système de commande d'endoscope à capsule comprenant un appareil de commande d'endoscope à capsule (11) conformément à la revendication 1 et un appareil de commande, communiquant avec l'appareil d'entraînement (2), et configuré pour fournir une interface de fonctionnement pour un utilisateur, pour transmettre une instruction de commande de mouvement de l'utilisateur vers l'appareil d'éntraînement (2), pour enregistrer et traiter des données visuelles recueillies par l'endoscope à capsule (7) à partir du corps humain.

8. Conformément à la revendication 7, le système de commande d'endoscope à capsule dans lequel la plaque arrière (302) est dotée d'un orifice de passage;
le palier (313) a une forme annulaire creuse;
dans laquelle, l'orifice de passage est mis en contact avec une position creuse du palier afin de déterminer une chambre de réception (303) pour héberger un candidat et le bras mobile (4) est placé sur un côté externe de la chambre de réception (303).

9. Conformément à la revendication 7, l'appareil de commande d'endoscope à capsule, dans lequel une plaque directrice est fixée de manière superposée sur un des côtés de la plaque arrière (302);
dans laquelle plaque directrice (314) est placée sur un anneau extérieur, sur le fond de la roulette entraînée (312); et
un bord extérieur de la plaque directrice (314) se prolonge en direction de l'un des côtés du bras mobile pour circonscrire un déflecteur circulaire (315) formant ainsi une partie protubérante (316) pointée vers le haut, dans une position proche de la roulette entraînée, sur un anneau interne de la plaque directrice (314);
le déflecteur (315), la plaque directrice (314) et la partie protubérante (316) délimitent une rainure directrice annulaire (317);
la rainure directrice (317) est dotée à l'intérieur, d'un câble de raccordement (401) au bras mobile (4).

10. Conformément à la revendication 7, l'appareil de commande d'endoscope à capsule, dans lequel une plaque d'installation (318) est fixée sur la surface supérieure de la roulette entraînée (312), et l'une des extrémités du bras mobile (4) est fixée sur la plaque d'installation (318).

11. Selon la revendication 7, l'appareil de commande de l'endoscope à capsule, dans lequel le bras mobile comprend:
une première tige de bras (411) dont l'une (411) des extrémités est fixée au dispositif de rotation (3);
une deuxième tige de bras (412) avec l'une des extrémités de la deuxième tige de bras (412) raccordée à l'autre extrémité de la première tige de bras (411) et l'autre extrémité de la deuxième tige de bras (412) étant raccordée à l'aimant permanent (5);
dans ce dispositif, un moteur est place séparément entre la première tige de bras (411) et le dispositif de rotation (3), entre la première tige de bras (411) et la deuxième tige de bras (412), et entre la deuxième tige de bras (412) et l'aimant permanent (5).

12. Conformément à la revendication 7, l'appareil de commande de l'endoscope à capsule, dans lequel le bras mobile (4) comprend:
une première tige de bras (411) dont l'une des extrémités est fixée au dispositif de rotation (3); une deuxième tige de bras (412) dont l'une (412) des extrémités est raccordée à l'autre extrémité de la première tige de bras (411); et
une troisième tige de bras (413), dont l'une (413) des extrémités est raccordée à l'autre extrémité de la deuxième tige de bras (412), et l'autre extrémité de la troisième tige de bras (413) étant raccordée à l'aimant permanent (5);
dans ce dispositif, un moteur est placé séparément, entre la première tige de bras (411) et le dispositif de rotation (3) entre la première tige de bras (411) et la deuxième tige de bras (412) et entre la deuxième tige de bras (412) et la troisième tige de bras (413).

13. Conformément à la revendication 7, le système de commande d'endoscope à capsule dans lequel le dispositif de commande comprend :
un module de réception des instructions (111), configuré pour transmettre le flux entrant des instructions de commande des mouvements par l'utilisateur vers le dispositif d'entraînement (2);
un module de réception de données (113), configuré pour recevoir les données visuelles recueillies et le flux sortant provenant par l'endoscope à capsule (7) à partir du corps humain;
un module de mémorisation de données (113), configuré pour mémoriser les données visuelles reçues par le module de réception de données et un module de traitement des données (114) configuré pour traiter, selon les fonctions activées par l'utilisateur, les données visuelles mémorisées par le module de mémorisation de données (113).
